# EUROPEAN PATENT APPLICATION

(11) **EP 3 210 619 A2**
(43) Date of publication of application: **30.08.2017**
(21) Application number: 15805257.1
(22) Date of filing: 26.10.2015
(51) Int. Cl.: A61K 38/48, A61K 9/06, A61K 47/14, A61P 31/00

(54) **TOPICAL FORMULATION FOR TREATING SKIN OR MUCOSAL INFECTIONS, PREPARATION METHOD AND USES THEREOF**

(30) Priority: 24.10.2014 PT 10800414
(71) Applicant: HPRD-Health Products Research and Development LDA, 6200-281 Covilhã (PT)
(72) Inventor: PALMEIRA DE OLIVEIRA, Rita Manuela, P-6200 Covilhã (PT); PALMEIRA DE OLIVEIRA, Ana Cristina, P-6200-590 Peraboa (PT)
(74) Representative: Patentree, Lda.
(86) International application number: PCT/IB2015/058245
(87) International publication number: WO 2016/063265

(57) **Abstract**

The present disclosure relates to the field of therapeutics for skin and mucosal infections and refers to the use of bromelain either alone or with antimicrobial agents to inhibit, to reduce or to treat biofilms in infections derived from the presence of this pathogenicity mechanism. This application aims to use the enzymatic action of bromelain to destroy the biofilms and allow for the penetration of the antifungals therefore improving their action in the site of infection, treating and reducing symptoms.

The present disclosure may be used in the pharmaceutical field for the treatment of infections, as creams, lotions, gels or vials for local application in the skin or mucosa.

Formulations with the ability to maintain the stability of the enzyme and of the other active components were developed with good characteristics for skin or mucosal application, particularly in the vagina, promoting, in this way, the efficacy and acceptability of the final product.

## Description

### Technical field

The present disclosure relates to the field of therapeutics for skin and mucosal infections and refers to the use of bromelain either alone or associated with antimicrobial agents to inhibit, to reduce or to treat biofilms in infections derived from the presence of this pathogenicity mechanism. This application aims to use the enzymatic action of bromelain to destroy the biofilms and allow for the penetration of the antifungals therefore improving their action in the site of infection, treating and reducing symptoms.

The present disclosure may be used in the pharmaceutical field for the treatment of infections, as creams, lotions, gels, vials or pessaries for local application in the skin or mucosa.

Formulations with the ability to maintain the stability of the enzyme and of the other active components were developed with good characteristics for application in the skin and mucosa, particularly in the vagina, promoting, in this way, the efficacy and acceptability of the final product.

### Background

The treatment of infections caused by microorganisms which present the ability to organize themselves in biofilms is a field that lacks strategies to promote efficacy.

Biofilms are microbial cells communities inserted in a polymeric matrix produced by themselves, which adhere to biological or inert surfaces. This structure represents a pathogenicity factor by conferring high resistance to antimicrobial agents and to the protective action of the host immune cells, since it is a mechanism of protection to the penetration of molecules and cells. Therefore, infections caused by strains with the ability to form biofilm translate in a higher difficulty of treatment and eradication frequently leading to therapeutic failure.

Fungus (yeasts) from the genus *Candida* spp. are examples of microorganisms with the ability to produce biofilms and are responsible for superficial (skin, mucosa and nails) and systemic infections. Concerning mucocutaneous infections, vulvovaginal candidosis (VVC) is the most frequent one affecting around 75% of women at least once in their lifetime, and 50% of these women end up by presenting recurrent episodes. *Candida albicans* is the most frequently isolated strain, being reported in 70 to 90% of the clinical isolates. Concerning systemic infections it is observed that the majority is associated with biofilm formation on the surface of catheters, cardiac valves or other medical devices, which release cells for the blood stream allowing them to reach other sites therefore disseminating the infection.

The first step for colonization is the adhesion of yeasts to the hosts' cells and/or medical devices, being the adhesion a central process for the formation of biofilms. It is estimated that 80% of human infections come from pathogenic biofilms.

The increased resistance to antibiotics expressed by cells in the biofilm represents one of the main recognized mechanisms of these organized structures.

Antifungal drugs from the azole therapeutic group (fluconazole, clotrimazole, itraconazole, etc.) are the first line option for the treatment of candidosis. The therapeutic options for the treatment of fungal infections are scarce, and the excessive use of these drugs (sometimes in subtherapeutic dose) favours the development of resistances, resulting in therapeutic failure. Azoles are even less effective in the presence of biofilms due to the diverse mechanisms of resistance presented by them.

Alternatives to conventional treatments have been proposed to increase the efficacy of treatments and reduce their recurrence. In this context, it has been shown that essential oils obtained from species of the genus *Thymus* spp. such as *Thymbra capitata* and *Thymus vulgaris* present antifungal activity, particularly anti-Candida activity including strains isolated from cases of recurrent infections.

The use of combined therapies arises as an attempt to overcome the problems inherent to microbial resistance. This strategy presents advantages that include the possibility of reducing the emergence of resistant strains and reduce the dose-related toxicity since, in combined therapies, the administered doses are usually lower for each drug, besides the intention to achieve a synergic effect among the combined compounds.

Bromelain is an enzyme isolated from the pineapple (*Ananas comosus*)*.* This fruit was extensively used by native cultures due to its medicinal properties which are currently attributed to bromelain. Bromelain is known for its reversible platelet aggregation inhibitory activity, antiedema, anti-inflammatory, antithrombotic and fibrinolytic properties. Bromelain also presents the ability to improve tissue permeability to antibiotics such as penicillin and tetracycline, following oral administration, that way improving the absorption and diffusion of antibiotics after subcutaneous and intramuscular administration. This enzyme is associated with very few adverse effects being, therefore, considered a well-tolerated drug.

To date bromelain has not been used alone to improve the treatment of infections through the changes caused in biofilms.

The document US2011182874A1 describes the use of an enzymatic system that mandatorily includes the peptidase from *Serratia* spp., and that may or may not be associated with other enzymes such as bromelain and papain, to be used in microbial biofilms. However, this patent does not advocate the use of bromelain as a single enzyme for this function referring in all claims the mandatory association with the peptidase from *Serratia* spp. and therefore, describing a multiple enzyme system. In the present disclosure it is shown that bromelain, not associated with any other enzyme, has the ability of reducing biofilms from *Candida* spp. being able to be applied in combination with azoles or plant extracts for the treatment of infections caused by this microorganism.

According to the Portuguese Pharmacopeia (FP IX), vaginal preparations include liquid preparations, semi-solids or solids intended to be administered through the vaginal route, usually to achieve a local action. They contain one or more active substances in an appropriate excipient. Several categories of vaginal preparations are distinguished: pessaries; vaginal tablets; vaginal capsules; solutions, emulsions or vaginal suspensions; tablets for vaginal solutions or suspensions; semi-solid vaginal preparations; vaginal foams and vaginal medicated tampons.

In practice, the dosage forms more frequently administered through the vaginal route are pessaries, tablets and ointments. In a recent study from our research team it was shown that Portuguese women's preferences regarding products for vaginal application relates to semi-solid dosage forms (gels, ointments, creams) confirming the international data from the literature previously published regarding contraceptives and vaginal microbicides. Additionally, women identify, as frequent disadvantages from vaginal preparations, the reduced retention time, due to the vaginal self-cleaning mechanism, being associated with discomfort and leakage; discomfort during application and pain or difficulty of adaptation to the body after application. These data are important to allow for the development of formulations for vaginal application which may be able to gather user's acceptability and, consequently, higher compliance to therapeutics with efficacy results.

These documents show the technical problem which is addressed by the present solution.

### General description

The present disclosure relates to the field of the treatment of infections and refers to the application of bromelain (an enzyme of natural origin used in therapeutics as an anti-inflammatory) or bromelain and antimicrobials (such as azoles, classical antifungals largely used in therapeutics or substances of natural origin, such as essential oils from *Thymbra capitata, Thymus* spp. which have been proposed as alternatives to conventional treatments) or bromelain alone to inhibit, reduce or treat biofilms in skin or mucosal infections derived from the presence of this pathogenicity mechanism. This application aims to use the enzymatic activity of bromelain to destroy infections caused by biofilms which affect the skin or mucosa and allow for the penetration of antifungals, therefore improving their action.

The present disclosure describes a formulation comprising bromelain in a dosage form appropriate for skin or mucosal application, particularly for vaginal application, which presents several challenges particularly because its rapid inactivation in aqueous media has been described. In fact, the products that are commercially available for oral use and that contain this enzyme are formulated as tablets which anhydrous composition allows for the maintenance of the substance stability throughout time. The present disclosure developed formulations which avoid bromelain degradation allowing for the maintenance of its efficacy in the treatment of skin and mucosal infections.

It has been demonstrated that, surprisingly, bromelain (not associated) has the ability to reduce the biomass of biofilms from *Candida* spp. It has also been demonstrated the reducing effect of the association of bromelain and clotrimazole regarding the biomass and metabolic activity of *C*. *albicans* biofilms. The antifungal effect the essential oils obtained from *Thymbra capitata, Thymus vulgaris* and other species from the *Thymus* genus was also shown.

Another aspect of the present disclosure describes semi-solid formulations with the ability to maintain the stability of the enzyme and of the remaining active components with good characteristics for skin and mucosal application, particularly in the vagina, that way promoting efficacy and users' acceptability of this novel therapeutic use as final product.

On the other hand, the mixture of the active substances with excipients with diluent, binder and lubricant functions allows for the formulation of tablets by the technique of compression and its inclusion in basis of pessaries allows its delivery as pessaries.

It has been shown, experimentally, that bromelain has the ability to promote the destruction of components from the biofilm matrix, allowing for the reduction of the biofilm biomass. It has also been observed that the combination of bromelain and clotrimazole markedly reduces the biomass and metabolic activity of biofilms from C. *albicans* strains. The combination of these substances always results in a higher effect than the sum of the individual activities of the components, resulting in the increased susceptibility of the yeasts to clotrimazole activity. Bromelain is, therefore, presented for an innovative use. The application of this combination, or of bromelain alone, in therapeutics must consider the acceptability profile of the users.

The present disclosure describes the application of the combination of drugs or bromelain alone with the goal of improving the treatment of infections through the inhibition, reduction or destruction of biofilms and promotion of the antimicrobial activity.

One of the surprising aspects of the present invention is the use of bromelain for the treatment of skin or mucosal infections, namely the effect of bromelain alone, that is, without being mixed with other enzymes. Furthermore, formulations were developed with improved effects for vaginal application of this enzyme alone or combined with other active substances characterized by reduced aqueous solubility. The studies performed to date show that this combination results in an enhancement of the antimicrobial final activity and not only on the sum of the individual activities of the drugs and that these activities are maintained in the final formulation.

The present disclosure relates to the application of bromelain and antimicrobials (such as azoles, classical antifungals or natural extracts with antimicrobial activity such as essential oils) for the treatment of infections caused by microorganisms which form biofilms as mechanism of pathogenicity. The innovation consists on the use of bromelain with the goal of inhibit, reduce or treat the biofilms due to its proteolytic action associated with the antimicrobial activity of substances of synthetic or natural origin which act through mechanisms of death or microbial growth inhibition. This combination aims at using the enzymatic activity of bromelain to destroy the biofilms and promote the penetration of the antifungals, enhancing their action. On the other hand, bromelain may be applied alone for the same goal, whenever there is no need to be combined with an antimicrobial.

This disclosure may be applied to the treatment of diverse skin or mucosal infections, particularly in the vagina, preferably for the treatment of mucocutaneous or systemic candidosis.

One of the aspects of the present disclosure relates to a topical formulation which comprises a therapeutically effective amount of the enzyme bromelain and an adequate excipient for the treatment of skin or mucosal infections, particularly in the vagina, particularly infections caused by microbial biofilms, namely mucocutaneous or systemic candidosis.

Bromelain is an enzyme of natural origin, derived from the pineapple, which is approved as an anti-inflammatory for oral use in the therapeutic field. Its application, not combined with other enzymes, for the treatment of infections with the goal of destroying the biofilms, had not been previously reported. The combination of this enzyme with antimicrobial drugs, particularly antifungals (azoles) used for the treatment of vulvovaginal candidosis and the natural extracts such as the example of essential oils from *Thymbra capitata* and from other species belonging to the genus *Thymus* is the second innovative aspect of this disclosure. In an embodiment, the application of these substances to the vagina demands an adequate formulation since, in one hand, the bromelain enzyme undergoes hydrolysis easily loosing activity in aqueous media while, on the other hand, the oil raises formulation challenges due to its low solubility in water. In this disclosure semisolid formulations were developed with non-aqueous base for vaginal application which overcome the mentioned stability limitations assuring, simultaneously, the acceptability of the users. The delivery of the same substances in solid dosage forms is also included as a variation of the formulation. Since vulvovaginal candidosis is a highly prevalent infection with high rates of recurrence and since biofilm formation is recognized as a factor associated with difficulties in treatment of the most difficult cases, this disclosure arises as a proposal of alternative treatment with potential efficacy for its treatment. Bromelain alone or in combination with antimicrobials may also be applied to other infections characterized by the presence of biofilms as long as it is correctly delivered in formulations, medical devices or wound dressing material.

An embodiment of the present disclosure relates to a topical formulation that may comprise 0.125-10% (w/w) bromelain, preferably 0.25-2% (w/w) bromelain, more preferably 0.25-1% (w/w) bromelain.

An embodiment of the present disclosure relates to a topical formulation that may comprise one of the following excipients: propylenoglycol, gelified propylenoglycol, carbopol, poliethyleneglycols and mixtures thereof.

An embodiment of the present disclosure relates to a topical formulation for the treatment of skin infections, particularly infections caused by bacterial biofilms which may comprise: 70-98% (w/w) de triacetin, 0-17% (w/w) de propyleneglycol; 0-17% (w/w) de glycerine; 1-11% (w/w) colloidal silicon dioxide; 0.125-10% (w/w) bromelain.

An embodiment of the present disclosure relates to a topical formulation that may comprise 7.5 -15 % (w/w) propyleneglycol and 0-5% (w/w) glycerine.

An embodiment of the present disclosure relates to a topical formulation that may also comprise a second antimicrobial agent.

An embodiment of the present disclosure relates to a topical formulation in which the concentration of the second antimicrobial agent may vary between 0.1-10% (w/w).

An embodiment of the present disclosure relates to a topical formulation in which the antimicrobial agent may be selected from *Thymus,* particularly *Thymbra capitata, Thymus vulgaris, Thymus mastichina* and mixtures thereof.

An embodiment of the present disclosure relates to a topical formulation in which the concentration of *Thymus* may vary from 0,1 - 1% (w/w), preferably 0,25 - 1% (w/w).

An embodiment of the present disclosure relates to a topical formulation in which the antimicrobial agent may have an antifungal activity.

An embodiment of the present disclosure relates to a topical formulation in which the antimicrobial agent may belong to the azole group.

An embodiment of the present disclosure relates to a topical formulation in which the concentration of the antifungal may vary between 0.5-3% (w/w), preferably 0,5-1% (w/w).

An embodiment of the present disclosure relates to a topical formulation in which the antifungal is clotrimazole.

An embodiment of the present disclosure relates to a topical formulation which may also comprise an antibiotic, preferably in a concentration varying between 1 - 10% (w/w).

An embodiment of the present disclosure relates to a topical formulation in which the antibiotic may be fusidic acid, bacitracin, neomycin, or combinations thereof.

In an embodiment it was developed semi-solid formulations of anhydrous base combining triacetin in a w/w concentration ranging from 70 to 98%, propyleneglycol (0-17%) or glycerin (0-17%) and aerosil 200 (1-11%) as gelifying agent. Formulations with different concentrations of excipients were prepared through dispersion of aerosol in approximately half the mixture of triacetin+humectant (propylenoglycol or glycerine) and through dissolution or dilution of the antifungal (azole or essential oil) in the remaining volume. After formation of the gel originated by the first mixture, the mixture of the antifungal with the previous is performed and homogenized to obtain the final preparation. This gel may be applied directly (in the vagina or affected skin) or be delivered through medical devices, wound dressing material or surgery material.

Another aspect of the present disclosure relates to a topical formulation to be used in the treatment of gynaecological infections, particularly vaginal infections, particularly the treatment of vulvovaginal candidosis.

Another aspect of the present disclosure relates to a gel, cream, lotion, vial, vaginal tablet or pessary which comprises the topical formulation described in the present disclosure.

Another aspect of the present disclosure relates to the use of bromelain as antimicrobial coating of articles, in which the article may be a medical device, a catheter, a prosthesis or a microchip.

The application of the combination of these drugs or of bromelain alone, in the therapeutic concentration, may be performed through formulations designed for each route of administration, comprising, at least one excipient of adequate pharmaceutical grade, with the ability to maintain the stability of the active substances and promote their efficacy. It can also be applied in medical devices and wound dressing material.

In an embodiment, a daily dose consists of a vial or other device, which comprises bromelain, which is to be administered, as a whole, as a single dose.

Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are for illustrating the specification and should not be seen as limiting the scope of the disclosure. Furthermore, this disclosure covers all possible combinations of particular or preferential embodiments herein described.

### Brief description of the drawings

The following figures provide preferred embodiments for illustrating the description and should not be seen as limiting the scope of the disclosure.
**Figure 1**: Graphical representation of the effect of bromelain concentration upon the biomass of biofilms from *Candida* spp. strains, wherein (Fig. 1.1) relates to collection strains (ATCC) and clinical strains of *C*. *albicans*; (Fig. 1.2) relates to clinical strains of *C*. *glabrata*; (Fig. 1.3) relates to clinical strains of *C*. *parapsilosis*; (Fig. 1.4) relates to clinical strains of *C*. *tropicalis*; (Fig. 1.5) relates to clinical strains of *C*. *guilliermondi*; and (Fig. 1.6) relates to clinical strains of *C*. *krusei.*
**Figure 2**: Graphical representation of the combination effect of bromelain and the antifungal clotrimazole upon the biomass of biofilms from *C*. *albicans* strains.
**Figure 3**: Graphical representation of the combination effect of bromelain and the antifungal clotrimazole upon the metabolic activity of biofilms from *C*. *albicans* strains.
**Figure 5**: Image representation obtained through confocal microscopy in a checkerboard assay to evaluate the combination of bromelain and clotrimazole upon AP25A (I) strain.

### Detailed description

The present disclosure describes a formulation comprising bromelain in a dosage form appropriate for skin or mucosal application, particularly for vaginal application, which presents several challenges particularly because its rapid inactivation in aqueous media has been described. In fact, the products that are commercially available for oral use and that contain this enzyme are formulated as tablets which anhydrous composition allows for the maintenance of the substance stability throughout time. The present disclosure developed formulations which avoid bromelain degradation allowing for the maintenance of its efficacy in the treatment of skin and mucosal infections.

The present disclosure also describes the application of bromelain combined with classical antifungal drugs (such as clotrimazole) or with plant extracts, namely the essential oils from *Thymbra capitata, Thymus vulgaris* and other species from the genus *Thymus,* or used alone for the treatment of fungal infections characterized by biofilm formation, such as mucocutaneous candidosis. These substances are included in one semisolid formulation of anhydrous base which assures the stability of the enzyme and the dissolution of azoles or the mixture with the essential oils.

In an embodiment of the present disclosure, it was shown that bromelain alone has the ability to reduce the biomass of the biofilms from *Candida* spp, in skin or mucosal infections. It was also shown the reduction of biomass and metabolic activity of *C*. *albicans* biofilms caused by the combination of bromelain and clotrimazole. The antifungal effect of the essential oils obtained from *Thymbra capitata, Thymus vulgaris* and other species which belong to the genus *Thymus* has also been previously shown.

The described disclosure consists of the application of the association of bromelain to antimicrobials, or bromelain alone towards the inhibition, reduction or treatment of biofilms in bacterial or fungal infections derived from the presence of this pathogenicity mechanism, such as mucocutaneous candidosis, skin infections or catheter-related infections caused by S. aureus.

Laboratory assays were performed in pre-formed mature biofilms (48h) in 96 wells microplates, using colorimetric assays described in the literature to quantify the biofilm biomass (Crystal violet assay) and the metabolic activity of cells (XTT reduction assay).

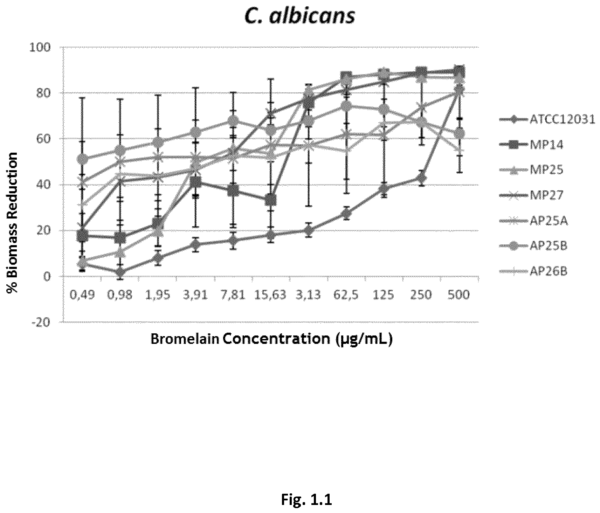

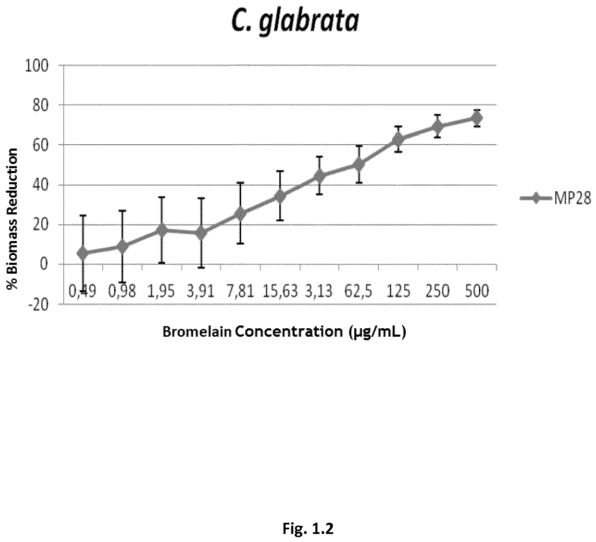

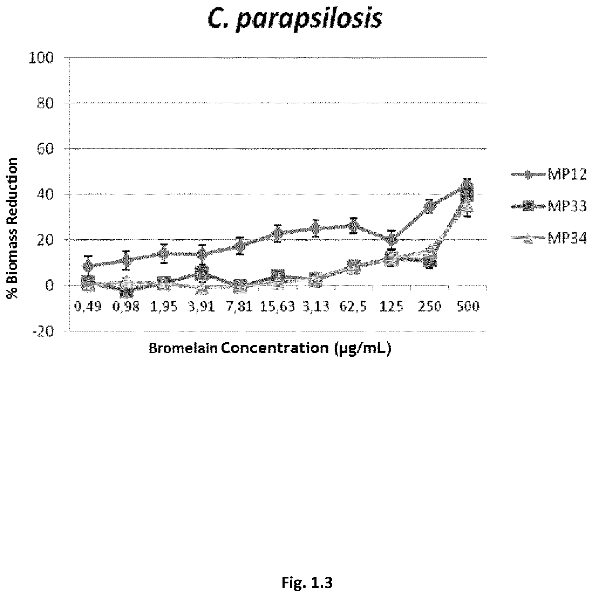

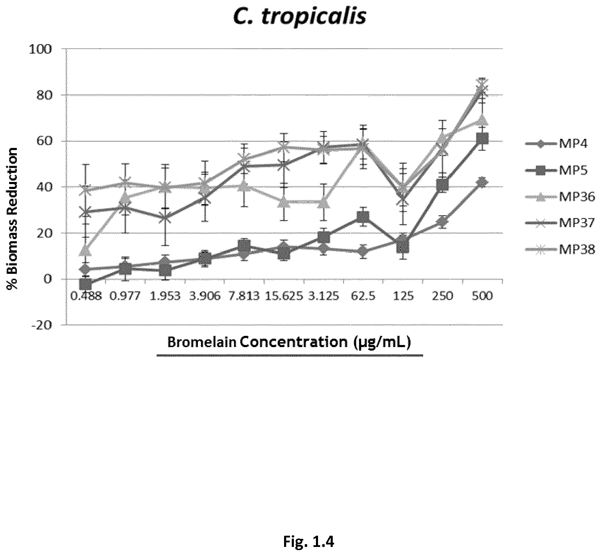

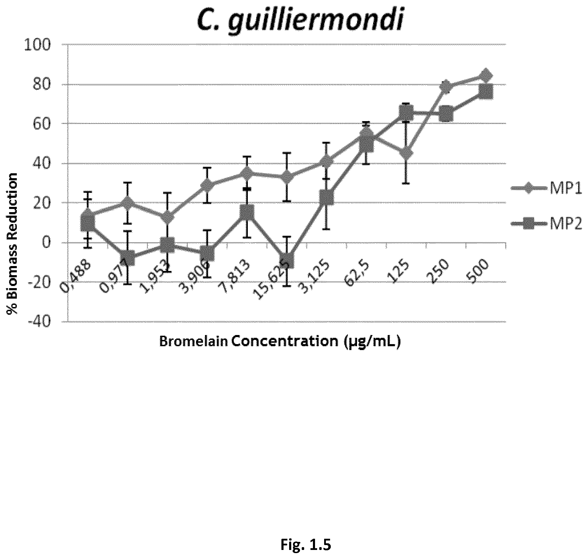

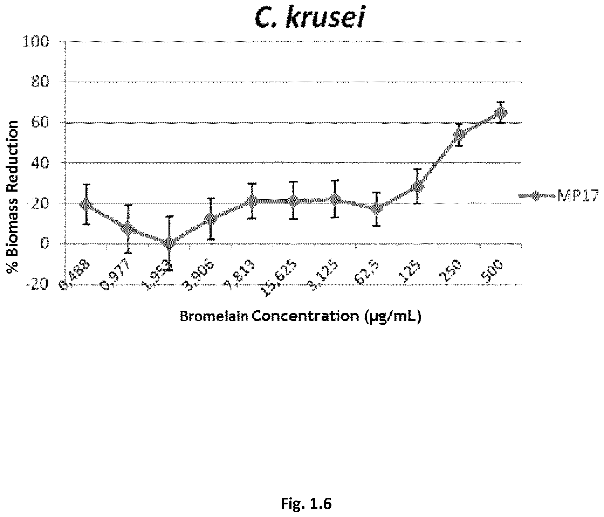

The effect of bromelain was tested in pre-formed biofilms of 20 Candida spp. strains belonging to 6 species: *C*. *albicans, C. glabrata, C. parapsilosis, C. tropicalis, C. guilliermondi* and *C*. *krusei.* Results are presented in Figure 1, as graphs showing the reduction of biomass of groups of strains according to their species. It was observed that the effect of bromelain alone upon the reduction of the biomass of Candida spp. biofilms occurs in all species, depending on the tested concentration and varying among strains. So, it is observed that the effect is not specific of a certain species under study. For the higher tested concentrations (250-500µg/mL) the reduction of biofilm biomass was achieved, varying from 40% to 80%, in all tested species and strains.

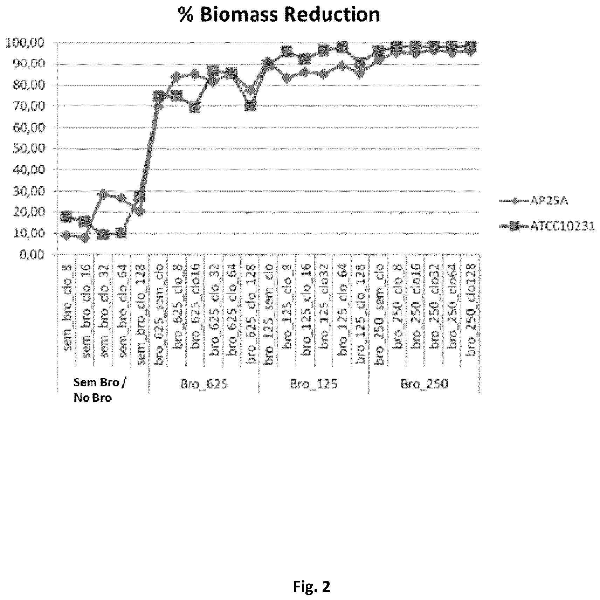

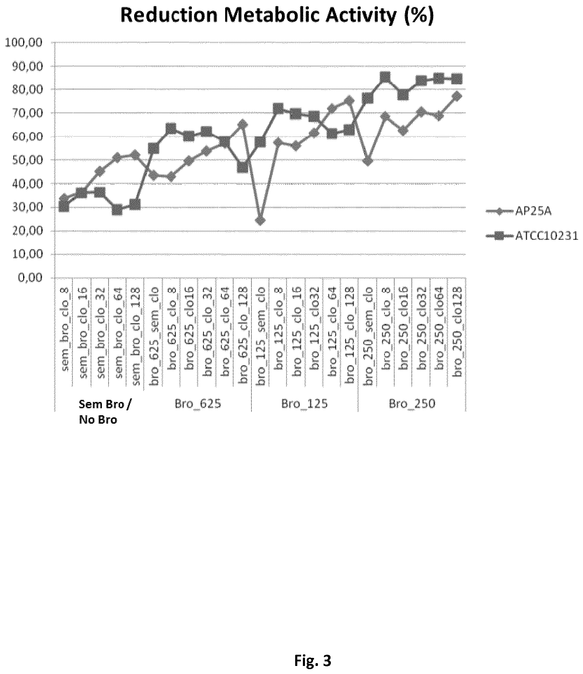

The combination effect of bromelain with the antifungal clotrimazole was evaluated through the checkerboard technique described by Roxana G. Vitale, Javier Afeltra and Eric Dannaoui in Antifungal Combinations and their results concerning biomass and metabolic activity reduction are represented in figures 2 and 3, respectively. Figure 2 shows that the effect of clotrimazole alone in the reduction of biofilm biomass is limited (without bromelain) and that the addition of the lowest tested concentration of bromelain causes a reduction of this parameter up to 80% for the tested C. albicans strains. For higher concentrations of bromelain this reduction even reaches 90%.

Concerning metabolic activity, figure 3 shows that increasing concentrations of clotrimazole cause reductions of activity in a concentration dependent manner that do not exceed 60% for the tested *C*. *albicans* strains. When bromelain is added, an increase of activity reduction is observed, dependent on the enzyme concentration (the addition of a 250 µg/mL bromelain solution leads to metabolic activity reductions near 80% for both strains). Previous studies undertaken by our team show that bromelain does not exhibit direct fungistatic or fungicidal activity against planktonic cells of different *Candida* species, including *C*. *albicans.* Thus, the increase in metabolic activity reduction shown for the combination of bromelain and clotrimazole is not due to a direct fungistatic activity of the enzyme, but to a degradation of the biofilm matrix. The degradation of this matrix allows for the penetration of clotrimazole in the biofilm favouring its antimicrobial action.

The anti-Candida activity of essential oils extracted from plants belonging to the genus *Thymus* had been previously shown.

Although bromelain does not present antifungal activity against planktonic cells, in this application the addition of bromelain, without clotrimazole, significantly reduces the metabolic activity, these reductions varying from 24.48% to 49.67% for the AP25A strains and 54.89% to 76.2% for ATCC 10231. The high reduction upon metabolic activity shown for bromelain are not due to a direct antifungal activity but to destruction of the biofilm, leading to the release of cells from the microplate. Following the washing step of microplates, the number of metabolically active cells available to metabolize XTT is reduced, leading to the overall reduction on metabolic activity.

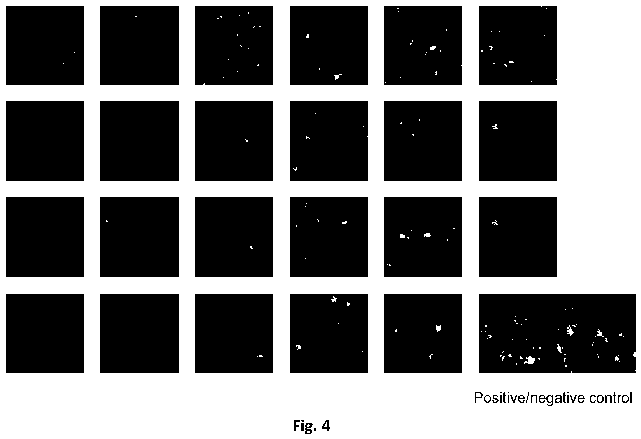

The images obtained by confocal microscopy, presented in figure 4, visually confirm the previously reported biomass reduction. By using calcofluor for the staining of biomass, a notorious effect from the combination of bromelain and clotrimazole throughout the biofilm can be seen, in comparison with control.

In an embodiment, formulations were developed as gels, deprived from water, for vaginal application of the combination of the antifungal with bromelain or of bromelain alone (table 1). These formulations contain excipients that mix with water presenting the advantage of being easily washable.

However, the fact that they do not contain water in their composition is a key factor to assure the stability of bromelain whose proteolysis and degradation in aqueous media has been shown in several studies. In an embodiment, one of the basis of formulations is triacetin, an excipient largely used in pharmaceutical, cosmetic and food industries. This excipient has been selected due to its reported safety not only following oral use but also due to its low irritation potential when applied with no dilution, on the skin and mucosa, as an occlusive patch (patch test). Safety studies performed with triacetin after application in the eye have shown that although it induces an ocular irritation it does not cause harm, supporting its safe use. On the other hand, concerning the vaginal route, this excipient is the basis of cream formulations approved to be used in the vagina and endocervical region, in Europe and the United States of America. Another humectant may be added to this excipient, such as propyleneglycol (PPG) or glycerin in concentrations ranging from 0 to 17% w/w. Colloidal silica dioxide (available under the trade mark Aerosil, among others, and used preferentially with 200 porosity) is used as a gelifying agent in concentrations ranging from 1 to 11% w/w allowing a gel to be formed with adequate viscosity and texture for vaginal application. Bromelain in concentrations of 0.125 to 10% w/w and/or the essential oil or the azole antifungal molecule in concentrations ranging from 0.1 to 3% w/w are dissolved or diluted on part of the mixture form by triacetin and PPG/glycerin or on triacetin (in case no PPG or glycerine is added) and mixed with the gel formed by Aerosil dispersion in the remaining vehicle.

**Table 1 - Examples of anhydrous gels containing bromelain and a natural or synthetic antifungal agent for the treatment of recurrent vulvovaginal candidosis**

| **Formulation** | **Raw material (name and concentration %w/w)** | | | | | |
|---|---|---|---|---|---|---|
| | Triacetin | Aerosil 200 | Propylenoglycol / Glycerina | Bromelain | *Thymbra capitata* essential oil | Clotrimazole |
| 1 | q.s. ad 100 | 8 | 15 | 1 | 1 | 0 |
| 2 | | 8 | 15 | 1 | 0 | 1 |
| 3 | | 8 | 0 | 1 | 1 | 0 |
| 4 | | 8 | 0 | 1 | 0 | 1 |
| 5 | | 8 | 0 | 0.25 | 1 | 0 |
| 6 | | 8 | 0 | 0.25 | 0.2 | 0 |
| 7 | | 8 | 7.5 | 1 | 1 | 0 |
| 8 | | 6 | 15 | 1 | 1 | 0 |
| 9 | | 6 | 0 | 0.25 | 1 | 0 |
| 10 | | 6 | 0 | 1 | 0 | 1 |
| 11 | | 6 | 0 | 0.25 | 0.17 | 0 |
| 12 | | 4 | 0 | 0.25 | 1 | 0 |
| 13 | | 4 | 0 | 1 | 1 | 0 |
| 14 | | 4 | 7.5 | 0.25 | 1 | 0 |
| 15 | | 4 | 7.5 | 0.25 | 0.17 | 0 |

Other formulations basis may be used to vehicle these agents such as propyleneglycol gelified with carbopol or polycarbophil and polyethyleneglycols.

Although in the present solution only particular embodiments have been represented and described, those skilled in the art will know how to modify or change the technical characteristics to equivalent ones, depending on the requirements of each situation, without departing from the scope of protection defined by the claims below.

The embodiments herein described are combinable.

The following claims set out particular embodiments of the disclosure.

## Claims

1. Topical formulation comprising a therapeutically effective amount of bromelain enzyme and an adequate excipient for the treatment of skin or mucosal infections, particularly infections caused by microbial biofilms.

2. Topical formulation according to the previous claim comprising 0.125 -10% (w/w) of bromelain.

3. Topical formulation according to the previous claim comprising 0.25-2% (w/w) of bromelain, preferably 0.25-1% (w/w) of bromelain.

4. Topical formulation according to the previous claims comprising also one of the following excipients: propyleneglycol, gelified propyleneglycol, carbopol, polyetheleneglycol and mixtures thereof.

5. Topical formulation for the treatment of skin infections, particularly infections caused by bacterial films comprising:
70-98% (w/w) triacetin,
0-17% (w/w) propyleneglycol;
0-17% (w/w) de glycerin;
1-11% (w/w) colloidal silica dioxide;
0.125-10% (w/w) bromelain.

6. Topical formulation according to the previous claims comprising 7.5 -15 % (w/w) of propyleneglycol and 0-5% (w/w) glycerin.

7. Topical formulation according to the previous claims comprising also an antimicrobial agent.

8. Topical formulation according to the previous claims, wherein the concentration of the antimicrobial agent ranges from 0.1 to 10% (w/w).

9. Topical formulation according to the previous claims, wherein the antimicrobial agent is selected among Thymus, particularly Thymbra capitata, Thymus vulgaris, Thymus mastichina, or mixtures thereof.

10. Topical formulation according to the previous claim, wherein the concentration of Thymus ranges from 0.1 to 1% (w/w), preferably 0.25 - 1% (w/w).

11. Topical formulation according to claims 7-10 wherein the antimicrobial agent has an antifungal activity.

12. Topical formulation according to claims 7-10 wherein the antimicrobial agent belongs to the azole group.

13. Topical formulation according to the previous claim, wherein the concentration of the fungicidal ranges from 0.5-3% (w/w), preferably 0.5-1% (w/w).

14. Topical formulation according to claims 8-9, wherein the fungicidal is clotrimazole.

15. Topical formulation according to the previous claims also comprising an antibiotic in a concentration ranging from 1 to 10% (w/w).

16. Topical formulation according to the previous claim, wherein the antibiotic is fusidic acid, bacitracin, neomycin, or combinations thereof.

17. Topical formulation according to the previous claims for use in the treatment of gynaecological infections, particularly vaginal infections.

18. Topical formulation according to the previous claim for use in the treatment of vulvovaginal candidosis or S. aureus.

19. Gel, cream, lotion, vial, vaginal tablet, or pessary comprising the topical formulation described in any of the previous claims.

20. Bromelain use as an antimicrobial coating composition for articles.

21. Use according to the previous claim wherein the article is a medical device, catheter, prosthesis or a microchip.
